# FASCICULE DE BREVET EUROPEEN

(11) **EP 1 103 618 B1**
(45) Date de publication et mention de la délivrance du brevet: **26.07.2006**
(21) Numéro de dépôt: 00403165.4
(22) Date de dépôt: 14.11.2000
(51) Int. Cl.: C12P 7/18

(54) **Procédé de purification du 1,3 propanediol a partir d'un milieu de fermentation**
Verfahren zur Reinigung von 1,3-propandiol aus einem Fermentationsmedium
Process for the purification of 1,3-propanediol from fermentation medium

(30) Priorité: 16.11.1999 FR 9914382
(43) Date de publication de la demande: 30.05.2001
(73) Titulaire: Roquette Frères, 62136 Lestrem (FR)
(72) Inventeur: Roturier, Jean-Michel, 59930 Chapelle D'Armentiere (FR); Fouache, Catherine, 62113 Sailly La Bourse (FR); Berghmans, Elie, 3071 Erps Kwerps (Kortenberg) (BE)
(74) Mandataire: Boulinguiez, Didier

(56) Documents cités:
- EP-A- 0 261 554
- EP-A- 0 361 082
- WO-A-96/35795
- CAMERON D C ET AL: "Metabolic engineering of propanediol pathways" BIOTECHNOLOGY PROGRESS, vol. 14, no. 1, 6 février 1998 (1998-02-06), pages 116-125, XP002067772
- TALMADGE K W ET AL.: "HPLC separations of a broad spectrum of small molecular weight analytes on cation-exchange columns" AMERICAN LABORATORY, vol. 29, no. 24, décembre 1997 (1997-12), pages 37-43, XP000922955
- SCHLIEKER H ET AL.: "Use of adsorption for product separation in the fermentation of glycerol to 1,3- propanediol;" CHEM.-ING.-TECH., vol. 64, no. 8, 1992, pages 727-728, XP002162858
- GÜNZEL B ET AL.: "Adsorption of diols from fermentation media on hydrophobic zeolites;" CHEM.-ING.-TECH., vol. 62, no. 9, 1990, pages 748-750, XP002162859
- BARBIRATO F ET AL.: "1, 3 - propanediol production by fermentation: An interesting way to valorize glycerin from the ester and ethanol industries" INDUSTRIAL CROPS AND PRODUCTS, vol. 7, no. 2-3, janvier 1998 (1998-01), pages 281-289, XP000990421

## Description

La présente invention est relative à un procédé de purification de 1,3 propanediol à partir d'un milieu de fermentation qui comprend du 1,3 propanediol et des coproduits de la fermentation.

La présente invention concerne plus particulièrement un procédé de purification de 1,3 propanediol par chromatographie sur résine cationique d'une solution aqueuse clarifiée issue du dit milieu de fermentation, de manière à obtenir au moins une fraction comprenant du 1,3 propanediol purifié, et au moins une fraction comprenant les coproduits de la fermentation.

Au sens de l'invention, on entend par « solution aqueuse clarifiée », la solution aqueuse obtenue après élimination des microorganismes producteurs de 1,3 propanediol du milieu de fermentation.

On entend également par « coproduits de la fermentation », les produits résultant d'un métabolisme secondaire tels que notamment le glycérol, ou le substrat non converti tel que par exemple le glucose ou le glycérol, ou encore des résidus divers provenant des éléments nutritifs du milieu de fermentation ou qui sont issus du métabolisme bactérien, tels que l'acide acétique, l'éthanol, ou le 2,3 butanediol.

La présente invention concerne plus particulièrement encore un procédé de purification du 1,3 propanediol par chromatographie, mettant en oeuvre une résine cationique dont le cation est avantageusement choisi dans le groupe constitué par le lanthane, le plomb, le zinc, le fer et l'aluminium.

La présente invention est enfin relative à un traitement, par des microorganismes qui convertissent de manière spécifique le glycérol en 1,3 propanediol, de ladite solution aqueuse clarifiée et/ou des fractions renfermant respectivement du glycérol et/ou un mélange de glycérol et de 1,3 propanediol, obtenues par la séparation chromatographique.

Les méthodes de purification du 1,3 propanediol à partir d'un milieu de fermentation qui sont connues en toute généralité dans l'état de la technique reposent principalement sur la succession d'étapes consistant à extraire le 1,3 propanediol dudit milieu de fermentation par des solvants organiques et/ou à le distiller.

Les techniques de distillation conventionnelles habituellement mises en oeuvre dans ces procédés de purification nécessitent un apport d'énergie important qui se répercute sur le coût du produit final ainsi purifié. En effet, la température de distillation du 1,3 propanediol est de 214°C.

MALINOWSKI (par exemple dans son article de *Biotechnology Techniques,* 1999, 13, pp 127-130) a proposé un procédé d'extraction liquide-liquide par solvants organiques du 1,3 propanediol, à partir de solutions aqueuses diluées le contenant.

Cependant, ce procédé de purification nécessite la manipulation de grandes quantités de solvants de type pentanol (jusqu'au nonanol), ou hexanal (jusqu'au décanal) et présente surtout une trop faible efficacité d'extraction et de séparation du 1,3 propanediol.

MALINOWSKI reconnaît d'ailleurs la nécessité d'avoir recours à une méthode alternative plus performante, le 1,3 propanediol présentant un caractère trop hydrophile.

Il a été également proposé comme alternative par BROEKHUIS et al. dans *Ind. Eng. Chim. Res.*, 1994, 33, pp 3230-3237, d'avoir recours à des composés chimiques de type formaldéhyde ou acétaldéhyde pour former un dérivé dioxolane du 1,3 propanediol, ce qui facilite alors son extraction par solvant.

Cependant, si le procédé dépense moins d'énergie, le coût des composés chimiques utilisés rend le procédé d'extraction et de purification rédhibitoire, puisqu'il faut en plus régénérer le 1,3 propanediol à partir de son dérivé dioxolane.

De la même manière, les procédés de complexation du 1,3 propanediol avec des organoborates proposés par BROEKHUIS et al dans *Ind. Eng. Chim.* Res., 1996, 35, pp 1206-1214, nécessitent d'importants moyens de régénération du 1,3 propanediol, ce qui rend ces procédés d'extraction non viables économiquement.

De tout ce qui précède, il résulte donc qu'il existe un besoin non satisfait de disposer d'un procédé simple à mettre en oeuvre, permettant de purifier le 1,3 propanediol à partir du milieu de fermentation d'un microorganisme producteur de 1,3 propanediol.

Soucieuse de développer un procédé qui permette de répondre mieux que ceux qui existent déjà aux contraintes de la pratique, la société Demanderesse a constaté qu'une purification efficace du 1,3 propanediol à partir dudit milieu de fermentation pouvait être réalisée en mettant en oeuvre un moyen consistant à réaliser une séparation chromatographique particulière des composants du milieu de fermentation.

Un procédé de séparation de 1,3 propanediol par chromatographie à partir d'un milieu de fermentation a bien été proposé par GÜNZEL et al (dans par exemple *DECHEMA BIOTECHNOLOGY Conferences,* 1990, 4 pt. B, pp 713- 716), consistant à mettre en oeuvre des techniques d'adsorption, et notamment une adsorption sur zéolites hydrophobes telles que la silicalite-1 ou les zéolites NaY désaluminées, voire du charbon actif.

Cependant les capacités d'adsorption du 1,3 propanediol sur ces supports sont très médiocres, et de plus il est déploré une fixation de certains des coproduits de la fermentation.

Le procédé de purification du 1,3 propanediol à partir d'un milieu de fermentation d'un microorganisme producteur de 1,3 propanediol comprenant du 1,3 propanediol et des coproduits de la fermentation constitués au moins de glycérol, conforme à l'invention est ainsi caractérisé par le fait que l'on :
a) sépare le micro-organisme producteur de 1,3 propanediol des autres composants du milieu de fermentation de manière à obtenir une solution aqueuse clarifiée,
b) passe ladite solution aqueuse clarifiée sur une résine échangeuse de cations, de manière à obtenir au moins une fraction comprenant du 1,3 propanediol purifié, et au moins une fraction comprenant les coproduits de la fermentation,
c) récupère le 1,3 propanediol.

Le microorganisme producteur de 1,3 propanediol est choisi dans le groupe des microorganismes recombinants capables par exemple de produire le 1,3 propanediol à partir de glucose, i.e. des microorganismes recombinants de type *E. coli* ou *S. cerevisiae* tels que décrits dans les demandes de brevet WO 96/35796 et WO 98/21341.

La société Demanderesse a remarqué que ces microorganismes produisent majoritairement d'une part du 1,3 propanediol et d'autre part notamment du glycérol comme coproduit de la fermentation. Il est également éventuellement retrouvé dans le milieu de fermentation du glucose qui n'a pas été entièrement consommé et un peu d'acide acétique.

La première étape du procédé conforme à l'invention consiste à séparer les microorganismes producteurs de 1,3 propanediol des autres composants du milieu de fermentation, de manière à obtenir une solution aqueuse clarifiée.

Cette séparation est réalisée par toute technique connue par ailleurs de l'homme du métier et peut consister en une microfiltration, une centrifugation ou une précipitation desdits microorganismes par des agents floculants. Ces techniques peuvent être également combinées.

Dans le procédé conforme à l'invention, il est préféré une technique de microfiltration, en utilisant une membrane de microfiltration dont la porosité est adaptée à la taille des microorganismes considérés, par exemple des membranes TECHSEP de 0,1 µm de porosité pour des microorganismes producteurs de 1,3 propanediol du genre *E. coli* recombinante.

On choisit avantageusement de passer la solution aqueuse clarifiée ainsi obtenue sur une colonne de charbon actif et de la déminéraliser sur une résine cationique forte et une résine anionique faiblement basique ou sur une résine cationique forte et une résine anionique fortement basique par exemple de type acrylique, en mettant en oeuvre les techniques connues en toute généralité de l'homme du métier.

Ces étapes permettent d'éliminer les débris cellulaires et les protéines résultant de la possible lyse des microorganismes producteurs de 1,3 propanediol, et le dessalage conduit à éliminer la charge minérale du milieu de fermentation.

On peut choisir ensuite de traiter ladite solution aqueuse clarifiée par un microorganisme qui convertit le glycérol résiduel en 1,3 propanediol.

Ce microorganisme est alors choisi dans le groupe constitué par des microorganismes des genres *Klebsiella, Citrobacter, Clostridium, Lactobacillus, Ilyobacter, Pelobacter* ou *Enterobacter* et est préférentiellement un *Citrobacter* ou un *Clostridium.*

Dans un mode de réalisation plus préférentiel du procédé conforme à l'invention, on choisit de mettre en oeuvre ce microorganisme capable de convertir le glycérol en 1,3 propanediol sous forme libre ou immobilisée, et de préférence sous forme immobilisée.

On peut réaliser cette immobilisation à l'aide d'alginate de calcium, tel que par exemple décrit dans le brevet us 5.164.309 pour des microorganismes du genre *Citrobacter,* ou utiliser la technologie développée par la société GENIALAB® BIO TECHNOLOGIE pour des microorganismes du genre *Clostridium* (communication au congrès ECB9 de Bruxelles, Juillet 1999, dans la session *Bioencapsulation processes*).

Cette étape de conversion du glycérol résiduel en 1,3 propanediol permet de ce fait d'augmenter le titre en 1,3 propanediol de la solution clarifiée, et ainsi de diminuer la composante glycérol des constituants de ladite solution aqueuse clarifiée, ce qui facilite d'autant la séparation chromatographique proprement dite.

La seconde étape du procédé conforme à l'invention consiste à passer la solution aqueuse clarifiée sur une résine échangeuse de cations.

Avantageusement, le cation est choisi dans le groupe constitué par le lanthane, le plomb, le fer, le zinc et l'aluminium.

Cette étape est effectuée de manière à obtenir au moins une fraction comprenant du 1,3 propanediol purifié, et au moins une fraction comprenant les coproduits de la fermentation.

Le mode de réalisation de cette séparation chromatographique est indifférent. Il est possible de choisir une séparation chromatographique discontinue, ou un procédé continu de séparation chromatographique.

Cette étape peut être avantageusement menée sur une résine échangeuse de cations fortement acide du type polystyrène acide sulfonique, réticulé par au moins 4 %, de préférence au moins 7 %, de divinylbenzène.

L'élution sera réalisée à l'eau, ce qui constitue une caractéristique importante du procédé de purification du 1,3 propanediol conforme à l'invention, car aucun solvant organique n'est ici à mettre en oeuvre. Le procédé est donc propre et satisfait aux normes de protection de l'environnement.

Dans un premier mode de réalisation du procédé conforme à l'invention, on préfère charger la résine avec un cation choisi dans le groupe constitué par le lanthane, le plomb et le fer.

La société Demanderesse a constaté que le choix de ces cations permet d'assurer une séparation efficace des composants de ladite solution clarifiée, comme il sera exemplifié ci-après, avec une élution de la fraction renfermant exclusivement le 1,3 propanediol dans les premières fractions de la chromatographie.

Dans un second mode de réalisation du procédé conforme à l'invention, on choisit un cation dans le groupe constitué par le zinc et l'aluminium.

La société Demanderesse a ici constaté que cette séparation chromatographique conduit à éliminer tous les coproduits de la fermentation du 1,3 propanediol dans les premières fractions d'élution, ce qui autorise la sortie dudit 1,3 propanediol dans les dernières fractions chromatographiques.

La société Demanderesse recommande, à cette étape de réalisation du procédé conforme à l'invention, de rassembler les éventuelles fractions contenant du glycérol comme coproduit de la fermentation, et de les soumettre à un traitement par les microorganismes capables de convertir le glycérol en 1,3 propanediol, de manière à obtenir une fraction enrichie en 1,3 propanediol.

Il est alors ensuite possible de rassembler cette fraction ainsi enrichie en 1,3 propanediol avec les fractions de 1,3 propanediol purifié obtenues au niveau de l'étape de séparation chromatographique mentionnées ci-dessus.

Il est toutefois préférable de réinjecter ladite fraction enrichie en 1,3 propanediol en amont de la séparation chromatographique pour en compléter la purification, si nécessaire.

La dernière étape du procédé conforme à l'invention consiste à rassembler toutes les fractions chromatographiques renfermant le 1,3 propanediol.

D'autres caractéristiques et avantages de l'invention apparaîtront à la lecture des exemples qui suivent. Ils ne sont toutefois donnés ici qu'à titre illustratif et non limitatif.

### EXEMPLE 1

Un milieu de fermentation renfermant 90,5 g/l de 1,3 propanediol, 28,7 g/l de glycérol, obtenu par fermentation d'une *E. coli* recombinante produisant du 1,3 propanediol à partir de glucose dans les conditions telles que décrites dans la demande de brevet WO 96/35796, est soumis à une microfiltration sur membrane TECHSEP de porosité 0,1 µm de manière à en éliminer les cellules.

La fermentation a été ici arrêtée lorsque tout le glucose introduit a été consommé par le microorganisme.

La solution aqueuse clarifiée ainsi obtenue renferme encore quelques débris cellulaires et une charge ionique de 70 meq/l.

Il est procédé à un passage sur colonne de charbon actif pour éliminer les protéines, suivi d'une étape de déminéralisation pour éliminer les ions résiduels du milieu de fermentation encore présents et ce, par passage sur résine cationique forte puis sur résine faiblement basique de type acrylique

La solution clarifiée ainsi déprotéinisée et dessalée présente une matière sèche de 12 % en poids.

Cette solution est ensuite soumise à une séparation chromatographique sur résine échangeuse de cations PCR 732 PUROLITE constituée de polystyrène acide sulfonique réticulé par 7 % de divinylbenzène, où le cation est du lanthane.

Cette résine étant initialement une résine sous forme H, on procède au remplacement du cation par percolation de la résine avec une solution de chlorure de lanthane à 200 g/l, jusqu'à ce que plus de 98 % de la capacité de la colonne soit sous la forme lanthane, ce qui est déterminé par le suivi du pH de la solution en sortie de colonne.

350 ml de cette résine sont alors placés dans une colonne de 2 m de haut, pour une section de 15 mm.

On introduit 2,3 ml de la solution clarifiée déprotéinisée et dessalée sur ladite colonne. L'élution est réalisée avec de l'eau introduite à un débit de 3 ml/min.

La température du système est fixée à 65 °C.

La détection des constituants des fractions collectées en sortie de colonne est réalisée par la mise en oeuvre d'un détecteur réfractomètrique R401 commercialisé par la société WATERS relié à une table tracante et leur analyse est réalisée par HPLC sur colonne BCX6 sous forme calcium à 6 % de DVB commercialisée par la société BIORAD, couplée à un détecteur réfractomètrique.

On obtient une première fraction de 39 ml contenant le 1,3 propanediol à 2,0 g/l (soit un rendement de 31,9 %, pourcentage exprimé par rapport à la quantité en poids de composés élués de la dite colonne).

Les deux autres fractions renferment respectivement :
- 51 ml d'un mélange de glycérol à 0,8 g/l et de 1,3 propanediol à 2,7 g/l (soit un rendement de 57,9 %),
- 50 ml de glycérol à 0,5 g/l (soit un rendement de 8,5 %).

Ces deux fractions ont été collectées, puis soumise à un traitement par des *Clostridium butyricum* NRRL B-1024 immobilisées sous forme LENTIKATS® , fournie par la société GENIALAB TECHNOLOGIE.

La conversion totale du glycérol a été conduite, permettant de rassembler toutes les fractions renfermant du 1,3 propanediol en une solution de haute pureté.

### EXEMPLE 2

La séparation chromatographique a été réalisée dans les mêmes conditions opératoires que l'exemple 1, mais avec une résine cationique dont le cation est le plomb.

On obtient une première fraction de 48 ml contenant le 1,3 propanediol à 3,0 g/l (soit un rendement de 47 %, pourcentage exprimé par rapport à la quantité en poids de composés élués de la dite colonne).

Les deux autres fractions renferment respectivement :
- 54 ml d'un mélange de glycérol à 0,7 g/l et de 1,3 propanediol à 1,4 g/l (soit un rendement de 37,5 %),
- 70 ml de glycérol à 0,5 g/l (soit un rendement de 11 %).

La conversion totale du glycérol a été conduite par des *Clostridium butyricum* NRRL B-1024 immobilisées sous forme LENTIKATS® , permettant de rassembler toutes les fractions renfermant du 1,3 propanediol en une solution de haute pureté.

## Revendications

1. Procédé de purification du 1,3 propanediol à partir d'un milieu de fermentation d'un microorganisme producteur de 1,3 propanediol comprenant du 1,3 propanediol et des coproduits de la fermentation constitués au moins de glycérol, **caractérisé par le fait que** l'on :
a) sépare le micro-organisme producteur de 1,3 propanediol des autres composants du milieu de fermentation de manière à obtenir une solution aqueuse clarifiée,
b) passe ladite solution aqueuse clarifiée sur une résine échangeuse de cations, de manière à obtenir au moins une fraction comprenant du 1,3 propanediol purifié, et au moins une fraction comprenant les coproduits de la fermentation,
c) récupère le 1,3 propanediol.

2. Procédé selon la revendication 1, **caractérisé par le fait que** le cation de la résine échangeuse de cation est choisi dans le groupe constitué par le lanthane, le plomb, le fer, le zinc et l'aluminium

3. Procédé selon l'une ou l'autre des revendications 1 et 2, **caractérisé par le fait que** la solution aqueuse clarifiée est passée sur une colonne charbon actif et déminéralisée sur une résine cationique forte et une résine anionique faiblement basique, ou sur une résine cationique forte et une résine anionique fortement basique

4. procédé selon l'une quelconque des revendications 1 à 3, **caractérisé par le fait que** la solution aqueuse clarifiée est traitée avec un microorganisme qui convertit le glycérol en 1,3 propanediol.

5. Procédé selon l'une quelconque des revendications 1 à 4, **caractérisé par le fait que** les fractions de chromatographie de l'étape b) comprenant notamment du glycérol comme coproduit de la fermentation sont traitées avec un microorganisme qui convertit le glycérol en 1,3 propanediol.

6. Procédé selon la revendication 5, **caractérisé par le fait que** les fractions ainsi traitées sont réintroduites en amont de l'étape de séparation chromatographique.

7. Procédé selon l'une quelconque des revendications 1 à 6, **caractérisé par le fait que** le microorganisme qui convertit le glycérol en 1,3 propanediol est choisi dans le groupe constitué par des microorganismes des genres *Klebsiella, Citrobacter, Clostridium, Lactobacillus, Ilyobacter, Pelobacter* ou *Enterobacter* et est préférentiellement un *Citrobacter* ou un *Clostridium*.

8. Procédé selon la revendication 7, **caractérisé par le fait que** le microorganisme convertissant le glycérol en 1,3 propanediol est mis en oeuvre sous forme libre ou immobilisée, et préférentiellement sous forme immobilisée.

## Claims

1. A process for the purification of 1,3-propanediol from a fermentation medium of a 1,3-propanediol-producing microorganism, the fermentation medium comprising 1,3-propanediol and fermentation coproducts consisting at least of glycerol, **characterized in that**:
a) the 1,3-propanediol-producing microorganism is separated from the other components of the fermentation medium so as to obtain a clarified aqueous solution;
b) said clarified aqueous solution is passed over a cation exchange resin so as to obtain at least one fraction comprising purified 1,3-propanediol and at least one fraction comprising the fermentation coproducts; and
c) the 1,3-propanediol is recovered.

2. Process according to claim 1, **characterized in that** the cation of the cation exchange resin is selected from the group consisting of lanthanum, lead, iron, zinc and aluminium.

3. The process according to any one of claims 1 and 2, **characterized in that** the clarified aqueous solution is passed over a column of activated carbon and demineralized over a strong cationic resin and over a weakly basic anionic resin, or on a strong cationic resin and a strongly basic anionic resin.

4. The process according to any one of claims 1 to 3, **characterized in that** the clarified aqueous solution is treated with a microorganism which converts the glycerol into 1,3-propanediol.

5. The process according to any one of claims 1 to 4, **characterized in that** the chromatographic fractions of step b) comprising in particular glycerol as fermentation coproduct, are treated with a microorganism which converts the glycerol into 1,3-propanediol.

6. The process according to claim 5, **characterized in that** the fractions thus treated are reintroduced upstream of the chromatographic separation step.

7. The process according to any one of claims 1 to 6, **characterized in that** the microorganism which converts the glycerol into 1,3-propanediol is selected from the group consisting of microorganisms of the genera *Klebsiella, Citrobacter, Clostridium, Lactobacillus, Ilyobacter, Pelobacter* and *Enterobacter*, and is preferably a *Citrobacter* or a *Clostridium.*

8. The process according to claim 7, **characterized in that** the microorganism converting the glycerol into 1,3-propanediol is employed in a free or an immobilized form, and preferably in immobilized form.

## Patentansprüche

1. Verfahren zur Reinigung von 1,3-Propandiol aus einem Fermentationsmedium eines 1,3-Propandiol produzierenden Mikroorganismus, das 1,3-Propandiol und Nebenprodukte der Fermentation enthält, die mindestens aus Glycerin bestehen, **dadurch gekennzeichnet, dass**:
a) der 1,3-Propandiol produzierende Mikroorganismus von den anderen Bestandteilen des Fermentationsmediums abgetrennt wird, um eine geklärte wässrige Lösung zu erhalten,
b) die geklärte wässrige Lösung über ein Kationenaustauscherharz geleitet wird, um mindestens eine Fraktion zu erhalten, die gereinigtes 1,3-Propandiol enthält, und mindestens eine Fraktion, die die Nebenprodukte der Fermentation enthält,
c) 1,3-Propandiol gewonnen wird.

2. Verfahren nach Anspruch 1, **dadurch gekennzeichnet, dass** das Kation des Kationenaustauscherharzes aus der Gruppe ausgewählt ist, die aus Lanthan, Blei, Eisen, Zink und Aluminium besteht.

3. Verfahren nach dem einen der Ansprüche 1 oder 2, **dadurch gekennzeichnet, dass** die geklärte wässrige Lösung über eine Aktivkohlesäule geleitet und an einem starken kationischen Harz und einem schwach basischen anionischen Harz oder einem starken kationischen Harz und einem stark basischen anionischen Harz entmineralisiert wird.

4. Verfahren nach einem der Ansprüche 1 bis 3, **dadurch gekennzeichnet, dass** die geklärte wässrige Lösung mit einem Mikroorganismus behandelt wird, der Glycerin in 1,3-Propandiol umwandelt.

5. Verfahren nach einem der Ansprüche 1 bis 4, **dadurch gekennzeichnet, dass** die Fraktionen der Chromatographie des Schrittes b), die insbesondere Glycerin als Nebenprodukt der Fermentation enthalten, mit einem Mikroorganismus behandelt werden, der Glycerin in 1,3-Propandiol umwandelt.

6. Verfahren nach Anspruch 5, **dadurch gekennzeichnet, dass** die so behandelten Fraktionen stromaufwärts des chromatographischen Trennungsschrittes wieder eingeführt werden.

7. Verfahren nach einem der Ansprüche 1 bis 6, **dadurch gekennzeichnet, dass** der Mikroorganismus, der Glycerin in 1,3-Propandiol umwandelt, aus der Gruppe ausgewählt ist, die aus Mikroorganismen der Gattungen *Klebsiella, Citrobacter, Clostridium, Lactobacillus, Ilyobacter, Pelobacter* oder *Enterobacter* besteht, und vorzugsweise *Citrobacter* oder *Clostridium* ist.

8. Verfahren nach Anspruch 7, **dadurch gekennzeichnet, dass** der Mikroorganismus, der Glycerin in 1,3-Propandiol umwandelt, in freier oder immobilisierter Form und vorzugsweise in immobilisierter Form eingesetzt wird.
